# EUROPÄISCHE PATENTSCHRIFT

(11) **EP 1 398 026 B1**
(45) Veröffentlichungstag und Bekanntmachung des Hinweises auf die Patenterteilung: **21.06.2006**
(21) Anmeldenummer: 03022406.7
(22) Anmeldetag: 11.02.1998
(51) Int. Cl.: A61K 31/12, A61P 11/06

(54) **Verwendung von 2,3-Dimethoxy-5-methyl-6-decaprenyl-1,4-benzochinon in der Behandlung von Inkontinenz**
The use of 2,3-dimethoxy-5-methyl-6-decaprenyl-1,4-benzoquinone in the treatment of incontinence
Utilisation de 2,3-dimethoxy-5-methyle-6-decaprenyle-1,4-benzoquinone dans le traitement de l'incontinence

(30) Priorität: 12.02.1997 DE 19705232
(43) Veröffentlichungstag der Anmeldung: 17.03.2004
(62) Teilanmeldung aus: 98908079.1
(73) Patentinhaber: MSE Pharmazeutika GmbH, 61348 Bad Homburg (DE)
(72) Erfinder: Enzmann, Franz, 61348 Bad Homburg (DE); Lachmann, Burkhard, 3000 DR Rotterdam (NL)
(74) Vertreter: Schreiber, Christoph

(56) Entgegenhaltungen:
- GB-A- 2 184 355
- US-A- 4 824 669
- US-A- 5 451 569
- DATABASE WPI Section Ch, Week 197814 Derwent Publications Ltd., London, GB; Class B05, AN 1978-26429A XP002266844 & JP 53 020432 A (EISAI CO LTD) 24. Februar 1978 (1978-02-24)

## Beschreibung

2,3-Dimethoxy-5-methyl-6-decaprenyl-1,4-benzochinon ist auch bekannt unter der Bezeichnung Coenzym Q10. Die Substanz spielt eine Rolle in der Atmungskette und ist obendrein ein Antioxidanz, welches in der Lage ist, Radikale, die insbesondere von Vitaminen weitergegeben werden, abzufangen und unschädlich zu machen. Q-10 bestimmt außerdem die Elastizität und die Dynamik der Zellmembranen. Es wird daher als Monopräparat und in Kombination mit anderen Wirkstoffen zur oralen Einnahme empfohlen. Zur Hautpflege wird es darüber hinaus in Form einer Liposomencreme angeboten, welche es dem Wirkstoff gestattet, durch die Hornschichtbarrieren einzudringen und sich dann in den verschiedenen Schichten der Haut anzureichern. Die bisher verwendete Liposomcreme wird hergestellt auf Basis von Lecithinen und bildet eine Lipiddoppelschicht um einen wässrigen Innenraum. Q-10 lagert sich dabei innerhalb der Membran an.

Es wurde jetzt gefunden, dass diese Substanz in weit größerem Umfang als bisher bekannt und vorherzusehen war, geeignet ist zur äußerlichen oder oralen Behandlung von Inkontinenz.

Zur oralen Anwendung kann es eingesetzt werden in Form von Pulver in Hartgelatinekapseln oder in öliger Suspension in Weichgelatinekapseln. Die Anmelderin vertreibt ein derartiges Monopräparat mit 30 mg des Wirkstoffes und auf ärztliche Anforderung mit 120 mg Q-10.

Zur äußerlichen Anwendung kann der Wirkstoff in Form von speziellen Liposomenzubereitungen zum Einsatz kommen. Liposome sind winzige kugelförmige Gebilde aus Lipidschichten und einem wässrigen Innenraum. Die Schichten entstehen durch geeignete Vermischung des Wirkstoffes mit Emulgatoren wie Lezithin. Q-10 lagert sich dabei direkt in die Lipiddoppelmembranen ein und dynamisiert sie dabei.

Anstelle der bisher verwendeten Liposomencreme kann auch eine Creme zur Anwendung kommen, welche zusätzlich eine wirksame Menge des Pulmonary Surfactant enthält. Liposome auf Basis des Pulmonary Surfactant sind im Gegensatz zu den zweischichtigen Liposomen aus Lecithin nur einschichtig. Diese Liposome sind in der Lage, noch rascher und intensiver in die Haut einzudringen. Die Kombination von herkömmlichen Liposomen und Pulmonary Surfactant erwiesen sich überraschenderweise also noch effektiver.

Der Pulmonary Surfactant ist ein aus den Lungen isolierbarer Komplex von speziellen Phospholipiden, neutralen Lipiden und Surfactant Proteinen, die miteinander eine einschichtige Barriere zwischen der Luft und der flüssigen Oberfläche der Lunge ausbilden. Der Pulmonary Surfactant wird in den Alveolaren Typ II Zellen gebildet und von dort in den Alveolarzwischenraum abgegeben. Der Pulmonary Surfactant kann auch aus den Komponenten rekombiniert werden.

Der Pulmonary Surfactant ist bisher nur eingesetzt worden zur Instillation bei Erkrankungen oder Mangelerscheinungen der Lunge.

Andere Anwendungen sind bisher nicht in Erwägung gezogen worden. Es wurde nun gefunden, dass Pulmonary Surfactant unerwarteterweise in die äußere Haut und die Schleimhaut des Magen-Darmbereiches, des Mund- und Vaginalbereiches einzudringen vermag, und zwar allein oder in Zusammenwirkung mit Liposomen.

Dabei spielt es eine untergeordnete Rolle, ob hochgereinigte oder weniger gereinigte Pulmonary Surfactant-Präparationen aller möglichen Spezies oder rekombinierter Pulmonary Surfactant herangezogen werden (Schwein, Rind, Schaf, etc). Weniger gereinigte Präparationen haben den Vorteil, dass sie sich kostengünstig produzieren lassen.

Pulmonary Surfactant ist somit geeignet, die Wirksamkeit von 2,3-Dimethoxy-5-methyl-6-decaprenyl-1,4-benzochinon zu verbessern und den Wirkungseintritt zu beschleunigen. Obwohl physiologischerweise Pulmonary Surfactant aus dem Lungengewebe abgegeben wird, vermag Pulmonary Surfactant überraschenderweise die Gewebsaufnahme von Pulmonary Surfactant und Q-10 zu steigern. Dies gilt in besonderem Maße für die jetzt gefundenen weiteren Indikationen zur Anwendung von 2,3-Dimethoxy-5-methyl-6-decaprenyl-1,4-benzochinon bei der Behandlung von Erkrankungen der Haut, der Schleimhäute und der Schleimhäute des Mundes, Magens und des Darmes sowie der Vagina.

Völlig neu sind die Wirksamkeit von Q-10 allein und in Kombination mit Pulmonary Surfactant bei der Behandlung von Inkontinenz.

## Patentansprüche

1. Verwendung von 2,3-Dimethoxy-5-methyl-6-decaprenyl-1,4-benzochinon zur Herstellung eines Arzneimittels zur Behandlung von Inkontinenz.

2. Verwendung nach Anspruch 1, **dadurch gekennzeichnet, dass** das Arzneimittel zusätzlich eine wirksame Menge des Pulmonary Surfactant enthält.

3. Verwendung gemäß Anspruch 2, **dadurch gekennzeichnet, dass** der Pulmonary Surfactant als Rohextrakt eingesetzt wird.

4. Verwendung nach Anspruch 1 bis 3, **dadurch gekennzeichnet, dass** das Arzneimittel Liposome enthält.

## Claims

1. Use of 2,3-dimethoxy-5-methyl-6-decaprenyl-1,4-benzoquinone for preparing a medicament for the treatment of incontinence.

2. The use according to claim 1, **characterized in that** said medicament additionally contains an effective amount of pulmonary surfactant.

3. The use according to claim 2, **characterized in that** said pulmonary surfactant is employed as a raw extract.

4. The use according to claim 1 to 3, **characterized in that** said medicament contains liposomes.

## Revendications

1. Utilisation de la 2,3-diméthoxy-5-méthyl-6-décaprényl-1,4-benzoquinone pour préparer des médicaments destinés au traitement de l'incontinence.

2. Utilisation selon la revendication 1, **caractérisée en ce que** les médicaments contiennent en plus une quantité active de surfactant pulmonaire.

3. Utilisation selon la revendication 2, **caractérisée en ce qu'**on utilise le surfactant pulmonaire sous forme d'extrait brut.

4. Utilisation selon la revendication 1 a 3, **caractérisée en ce que** les médicaments contiennent des liposomes.
